# EUROPEAN PATENT APPLICATION

(11) **EP 1 477 191 A1**
(43) Date of publication of application: **17.11.2004**
(21) Application number: 03703126.7
(22) Date of filing: 31.01.2003
(51) Int. Cl.: A61L 27/24, A61L 27/44

(54) **COMPOSITE BIOMATERIAL CONTAINING PHOSPHOLINE**

(30) Priority: 19.02.2002 JP 2002041409
(71) Applicant: National Institute of Advanced Industrial Science and Technology, Tokyo 100-8921 (JP)
(72) Inventor: UEMURA,Toshimasa, National Inst. of Adv. Ind. Sci., chome, Tsukuba-shi, Ibaraki 305-8566 (JP); TATEISHI, Tetsuya, Tsukuba-shi, Ibaraki 305-0042 (JP); SAITO, Takashi, Ebetsu-shi, Hokkaido 067-0062 (JP); IEJIMA, Daisuke, Kodaira-shi, Tokyo 187-0032 (JP)
(74) Representative: VOSSIUS & PARTNER
(86) International application number: PCT/JP2003/001004
(87) International publication number: WO 2003/070290

(57) **Abstract**

This invention relates to composite biomaterials having a sponge-like structure and comprising phosphophoryn and collagen, and to artificial bones obtained by culturing bone marrow-derived cells on the aforementioned composite biomaterials.

## Description

### Field of the Invention

The present invention relates to composite biomaterials comprising phosphophoryn and collagen, and to artificial bones obtained by culturing bone marrow-derived cells on the composite biomaterials.

### Background Art

Recently in the field of orthopedics, artificial bone graft is often used to repair bone defects. Such artificial bones are required to have biocompatibility and osteoinductivity in addition to mechanical properties similar to those of natural bones. That is, artificial bones need to be gradually resorbed after implantation in the body, become involved in the bone remodeling, and then be substituted for the natural bone.

A variety of materials, such as ceramic and organic materials, have been developed as materials for artificial bone. An example of a leading material that actively induces bone regeneration is a composite biomaterial comprising bone morphogenic proteins (BMP) and collagen. Other materials have low inducibility of bone regeneration. Although BMP is a potent osteogenic substance, it is less soluble in water. Moreover, a suitable carrier thereof has not yet been synthesized in spite of attempts to utilize collagen as a carrier. Also, the BMP has a high osteogenic ability for rats or mice, whereas a large amount of BMPs (as much as approximately 0.4 mg/ml (volume of the carrier)) is necessary for having effective osteogenesis for humans. Thus, the use of BMP is currently limited to the case of expensive medical care. Development of biodegradable materials for bone regeneration that are safe, inexpensive, osteogenic, and alternative to BMP is therefore desired.

The inventors have found that phosphoproteins contained in the teeth, such as phosphophoryn or phosvitin, have the osteogenic ability(Saito et al., Bone 21 (4), pp. 305-311, 1997), however, no report has been published concerning their application to bone regeneration or development of artificial bones.

Artificial bones are desired to be biodegradable and easily formed in order to suitably fill in bone defects with complex shapes. Type I collagen has a flexible structure and plays a key role in nucleation of hydroxyapatite. Thus, type I collagen is a desirable material for bone regeneration with three-dimensional structure. When it is combined with a porous hard material (for example, hydroxyapatite, β-TCP, or polylactic acid), it can be used as a high-quality implant or scaffold. Thus it is important to add an osteogenic ability to the collagen sponge, and development of a method that utilizes inexpensive osteogenic substances other than BMP is desired.

### Disclosure of the Invention

An object of the present invention is to provide artificial bones that are highly capable of osteogenesis (osteoinductive), biodegradable, easily formable, and cost-effective.

The present inventors have conducted concentrated studies in order to attain the above object. As a result, they have found that a composite material with a sponge-like structure in which a phosphoprotein "phosphophoryin" is crosslinked to type I collagen has the excellent capacity for osteogenesis. This has led to the completion of the present invention.

Specifically, the present invention provides the following (1) to (8).
(1) Composite biomaterials comprising phosphophoryn and collagen.
(2) The composite biomaterials according to (1), wherein the collagen is type I collagen.
(3) The composite biomaterials according to (1) or (2), wherein the phosphophoryn is crosslinked to collagen.
(4) The composite biomaterials according to any one of (1) to (3), which have sponge-like structures.
(5) The composite biomaterials according to any one of (1) to (4), which further comprise at least one selected from among hydroxyapatite, β-TCP, α-TCP, polyglycolic acid, and polylactic acid.
(6) The composite biomaterials according to any one of (1) to (5), which further comprise bone marrow-derived cells.
(7) Artificial bones comprising the composite biomaterials according to any one of (1) to (6).
(8) A method for producing composite biomaterials, wherein collagen fibers are crosslinked to phosphophoryn with divinylsulfone or 1-ethyl-3-(3-dimethylaminopropyl)carbodiamide, followed by lyophilization.

Hereafter, the present invention is described in detail.

### 1. Composite biomaterials

The composite biomaterials of the present invention comprise phosphophoryn and collagen as essential components. In such composite biomaterials, phosphophoryn is preferably crosslinked to collagen fibers. Also, the composite biomaterials preferably have a sponge-like microporous structure. This structure gives suitable properties as a scaffold for cell culture, which will be described below. The term "sponge-like structure" used herein refers to a flexible microporous structure in which a large number of approximately several-µm to several-10-µm pores (gaps) exist.

Phosphophoryn contained in the aforementioned composite biomaterials is also known to be contained in the teeth of mammals.

The collagen that is employed in the present invention is preferably type I collagen because type I collagen is a major component of bone and tooth organic matter and has high biocompatibility.

In the composite biomaterials of the present invention, the ratio of phosphophoryn mixed with collagen (weight ratio) is preferably in the range of 1:10 to 1:50, and more preferably in the range of 1:20 to 1:40. The amount of phosphophoryn added is preferably in the range of 2% to 10% (weight %) (hereafter "weight %" is simply referred to as "%"), and more preferably in the range of 2.5% to 5%, based on the total amount of the composite biomaterials of the present invention (total weight). This is because too little an amount of phosphophoryn results in an insufficient capacity for osteogenesis, and too great an amount thereof results in an increased cost of the composite biomaterials.

In the composite biomaterials of the present invention, the porosity is preferably in the range of 40% to 90%, and more preferably in the range of 60% to 90%. When the porosity is outside of this range, cell invasion becomes insufficient after implantation into the body, which in turn deteriorates osteoinductivity and the strength of the composite biomaterials.

In addition to essential components, i.e., phosphophoryn and collagen, the composite biomaterials of the present invention may comprise porous hard materials such as hydroxyapatite, β-TCP, α-TCP, polyglycolic acid, and polylactic acid within the scope of the present invention.

### 2. Method for producing composite biomaterials

### (1) Preparation of phosphophoryn

Commercially available phosphophoryn (e.g., that manufactured by Wako Pure Chemical Industries, Ltd.) may be used for the composite biomaterials of the present invention. Alternatively, it can be obtained, for example, in the following manner. Teeth of mammals (such as bovine) are extracted, and soft tissues, dental pulp, dental enamel, and dental cement are removed therefrom. The remaining dentin is finely pulverized, and the resultant is demineralized using a buffer containing a proteolytic enzyme (e.g., 0.5 M EDTA or 0.05 M Tris-HCl (pH 7.4)), followed by dialysis and lyophilization. Subsequently, the lyophilization product is dissolved in a buffer (e.g., 20 mM Tris-HCl, (pH 7.4, containing a proteolytic enzyme)), and calcium chloride is added. The resulting sediment is dissolved in a buffer (e.g., 0.5 M EDTA or 0.05 M Tris-HCl (pH 7.4, containing a proteolytic enzyme)), dialyzed, and then lyophilized again. Finally, the aforementioned lyophilization product is dissolved in a urea solution (e.g., 4 M urea, 0.01 M Tris-HCl (pH 8.0)), and then separated via ion exchange chromatography (e.g., DEAE-Sepharose) or other means. The phosphophoryn of interest can be identified via the phosphoric acid and amino acid analyses.

### (2) Purification of type I collagen

Collagen (type I collagen) that is employed in the present invention is not particularly limited. Commercially available collagen may be employed, or it may be extracted and purified from a suitable material containing collagen (e.g., the connective tissues of animals such as bovine dermis) in accordance with a conventional technique.

### (3) Preparation of composite biomaterials comprising phosphophoryn and collagen

Collagen fibers are first dissolved in an aqueous solution of carbonate such as potassium carbonate or sodium carbonate, and the solution is incubated at room temperature. The concentration of this aqueous carbonate solution is preferably 0.1 M to 0.2 M, and more preferably 0.4 M to 0.5 M. A crosslinking agent, such as divinylsulfone or 1-ethyl-3-(3-dimethylaminopropyl)carbodiamide, is added thereto, and a crosslinking bond is previously introduced onto collagen fibers. The amount of the crosslinking agent to be added is preferably about 5 weight % for divinylsulfone.

Subsequently, phosphophoryn is added, incubated, and then crosslinked to collagen. The amount of phosphophoryn to be added is preferably 1/10 to 1/50, and more preferably 1/20 to 1/40, relative to the amount of collagen (weight ratio). The resultant is washed with distilled water and then bicarbonate solution (e.g., sodium bicarbonate or potassium bicarbonate) and excessive amounts of phosphophoryn or crosslinking reagents are removed. Finally, sodium bicarbonate and mercaptoethanol are added to terminate the crosslinking reaction, and the product is thoroughly washed with distilled water, followed by lyophilization. Conditions for the aforementioned lyophilization (e.g., the temperature, freezing time, or lyophilization in water) can be adequately adjusted in accordance with, for example, the structure of the composite biomaterials of interest, i.e., the specific surface area, the porosity, the sizes of pores (void), and the like. The resulting lyophilized product can be shaped according to need and then used as, for example, artificial bones described below.

### 3. Artificial bones

### (1) Artificial bones comprising composite biomaterials

The composite biomaterials of the present invention have similar elasticity of sponges with water absorption and have excellent bioadaptability, osteoinductivity, and osteoconductivity. Specifically, when the composite biomaterials are implanted into bone tissues, they can become rapidly fused therewith, and the interface between the composite materials of the present invention and the hard tissues of the recipient can be completely integrated. Accordingly, the composite biomaterials of the present invention can be used as artificial bones for repairing and regenerating bone defects.

The configurations and shapes of the aforementioned artificial bones are not particularly limited. Artificial bones can take any desired configurations or shapes, such as sponges, meshes, unwoven fabric products, discs, films, sticks, particles, or pastes. These configurations and shapes may be suitably selected depending on the relevant applications.

### (2) Artificial bones comprising bone marrow-derived cells

The composite biomaterials of the present invention can be used as artificial bones that can be more effectively used for bone regeneration by inoculating bone marrow-derived cells thereto and conducting tissue culture in a biomimetic environment or *in vivo.*

The cells used for the artificial bones are undifferentiated cells with differentiation and proliferation ability. Examples thereof include mesenchymal stem cells, hematopoietic stem cells, skeletal muscle stem cells, neural stem cells, and hepatic stem cells. Bone marrow-derived ES (embryonic stem) cells and mesenchymal stem cells are particularly preferable. In addition to established cell lines, cells isolated from the body of a patient may also be used.

Cells transformed with growth factor genes may be cultured in accordance with a conventional technique in a bioadaptable scaffold. Cells may be simply inoculated into the bioadaptable scaffold, or inoculated in the form of mixtures with a liquid such as a buffer, physiological saline, a solvent for injection, or a collagen solution. When the cells do not smoothly enter into a pore because of the porous structure of the material, cells may be inoculated under low pressure.

Preferably, the number of cells to be inoculated (inoculation density) is adequately determined in accordance with the types of cells or scaffolds in order to reconstruct tissues more efficiently while maintaining the morphology of the cells. For example, the inoculation density is preferably 1,000,000 cells/ml or higher in the case of osteoblasts.

A conventional medium for cell culture, such as MEM medium, α-MEM medium, or DMEM medium, can be suitably selected depending on the type of cells to be cultured. FBS (Sigma), antibiotics such as Antibiotic-Antimycotic (GIBCO BRL), antibacterial agents, growth factors, transcription factors, or other substances may be added to the medium. Culture is preferably conducted in the presence of 3% to 10% CO₂ at 30°C to 40°C, and particularly preferably in the presence of 5% CO₂ at 37°C. The culture period is not particularly limited, and it is preferably 3 days or longer.

The thus-produced artificial bones can be implanted or injected into bone defects in the body of a patient.

### 4. Others

The composite biomaterials of the present invention can be used as scaffolds for other bioactive substances, drugs, and the like. For example, when the composite materials of the present invention adsorbed with anti-cancer agents are used for reconstructing bones removed due to osteogenic sarcoma, recurrence of the sarcoma can be prevented and the generation of hard tissue in an organism can be induced.

Accordingly, the composite biomaterials of the present invention can be utilized as, for example, materials capable of inducing bone regeneration and conducting bones, scaffolds for bioactive agents in tissue engineering containing amino acids, saccharides, and cytokines, and biocompatible drug carriers for sustained release. Specific examples of applications include artificial bones, artificial joints, cements for tendons and bones, dental implants, percutaneous terminals for catheters, drug carriers for sustained release, chambers for bone marrow induction, and chambers or scaffolds for tissue reconstruction.

### Brief Description of the Drawings

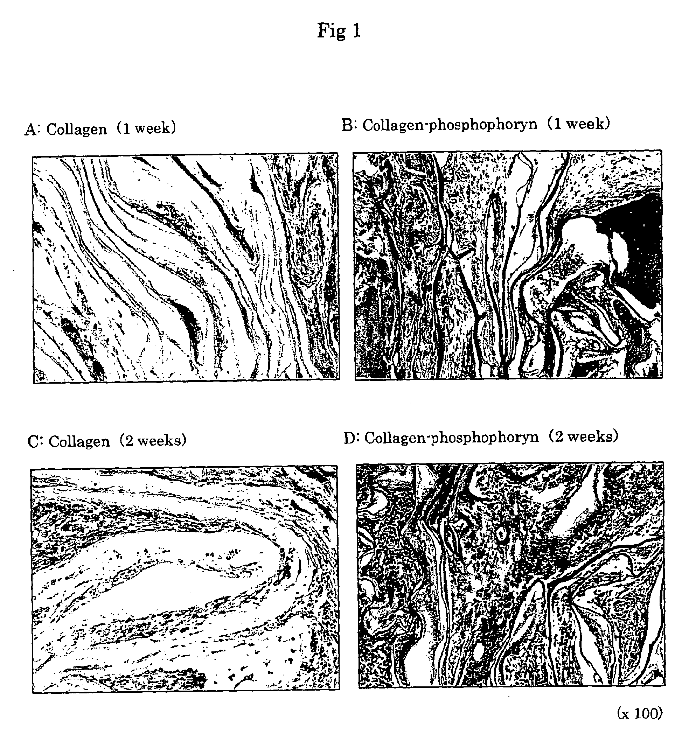

Fig. 1 is a photograph showing images of HE-stained tissues 1 and 2 weeks after implantation of the collagen-phosphophoryn or collagen sheets in Example 2: wherein A shows the case of collagen (1 week); B shows that of collagen-phosphophoryn (1 week); C shows that of collagen (2 weeks); and D shows that of collagen-phosphophoryn (2 weeks).

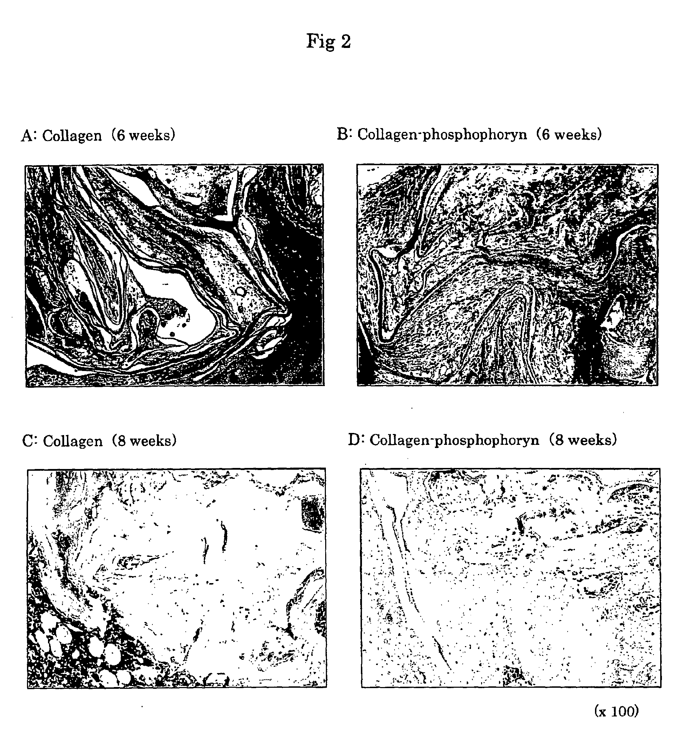

Fig. 2 is a photograph showing images of HE-stained tissues 6 and 8 weeks after implantation of the collagen-phosphophoryn or collagen sheets in Example 2: wherein A shows the case of collagen (6 weeks); B shows that of collagen-phosphophoryn (6 weeks); C shows that of collagen (8 weeks); and D shows that of collagen-phosphophoryn (8 weeks).

This description includes part or all of the contents as disclosed in the description of Japanese Patent Application No. 2002-41409, which is a priority document of the present application.

### Best Modes for Carrying out the Invention

The present invention is hereafter described in greater detail with reference to the examples, although the technical scope of the present invention is not limited thereto.

### Example 1: Production of composite biomaterials of phosphophoryn/collagen

### (1) Purification of phosphophoryn

At the outset, permanent teeth were extracted from the bovine lower jaw pyramid, and soft tissues, dental pulp, dental enamel, and dental cement were removed therefrom. The remaining dentin was finely pulverized to 200-mesh or smaller particles in liquid nitrogen. Dentin powder was demineralized using 0.5 M EDTA and 0.05 M Tris-HCl (pH 7.4, containing protease inhibitors: 100 mM 6-aminohexanoic acid (Wako Pure Chemical Industries, Ltd.), 5 mM benzamidine-HCl, and 1 mM phenylmethylsulfonyl fluoride) at 4°C.

Subsequently, the solution of demineralized EDTA was dialyzed with deionized and distilled water using a dialysis membrane (Spectrum MWCO 3500, 132725) at 4°C and then lyophilized (Eyela Freeze-Dryer 90500042, Tokyo Rikakikai Co., Ltd.). The EDTA extract was dissolved in 20 mM Tris-HCl (pH 7.4, containing a proteolytic enzyme), and CaCl₂ was added thereto to a final concentration of 1 M. The sediment was recovered via centrifugation (Himac Centrifuge 345043, Hitachi Koki Co., Ltd.), dissolved again in 0.5 M EDTA and 0.05 M Tris-HCl (pH 7.4, containing a proteolytic enzyme), dialyzed against deionized and distilled water, and then lyophilized. The lyophilized product was dissolved in 4 M urea and 0.01 Tris-HCl (pH 8.0) and then eluted with a linear gradient from 0 M to 1 M NaCl via column chromatography using DEAE-sepharose (Sigma Chem. Co.).

Finally, phosphophoryn was identified using the phosphoric acid and amino acid analyses.

### (2) Purification of type I collagen

Bovine skin was thinly sliced and washed with distilled water, 20% NaCl, and 0.05 M Tris-HCl (pH 7.4) at 4°C. Subsequently, extraction was carried out with the use of 1 M NaCl and 0.05 M Tris-HCl (pH 7.4) overnight, the supernatant was recovered by centrifugation, 0.5 M acetic acid and 1 M NaCl were added, and the mixture was agitated overnight.

The residue was recovered via centrifugation, dissolved in 0.5 M acetic acid, and then further centrifuged. The supernatant was neutralized with 5 M NaOH and 4.4 M NaCl, agitated overnight, and then centrifuged. NaCl (4.4 M) and 0.05 M Tris-HCl (pH 7.4) were added to this residue, and the resultant was agitated overnight, followed by centrifugation.

NaCl (2.4 M) and 0.05 M Tris-HCl (pH 7.4) were added to the residue, and the resultant was agitated overnight, followed by centrifugation. NaCl (1.7 M) and 0.05 M Tris-HCl (pH 7.4) were added to the residue, and the resultant was agitated overnight, followed by centrifugation. The obtained supernatant was dialyzed against 0.1 M acetic acid and then lyophilized.

The lyophilized collagen and 50 mM acetic acid were used to prepare a solution of 0.3% collagen in acetic acid. NaCl (0.15 M), 0.6 N NaOH, and 0.1 M Hepes (Wako Pure Chemical Industries, Ltd.) were added in that order, and the mixture was incubated at 37°C. Thus, type I collagen fibers were reconstructed.

### (3) Crosslinking of phosphophoryn to collagen

The collagen fibers obtained in (2) above were incubated using 0.5 M sodium carbonate at room temperature overnight. Divinylsulfone (Sigma Chem. Co.) was further added, and incubation was carried out for 2 hours. The collagen fibers were thoroughly washed with 0.5 M sodium carbonate, phosphophoryn was added, and the resultant was incubated overnight for crosslinking. The product was washed with distilled water, and thoroughly washed with 0.5 M sodium bicarbonate to eliminate excessive amounts of phosphophoryn and divinylsulfone.

Subsequently, 0.5 M sodium bicarbonate and mercaptoethanol were added, the resultant was incubated overnight, and the crosslinking reaction was terminated. The resulting composite was washed with distilled water, 0.5 M NaCl, 0.05 M Tris-HCl (pH 7.4), and distilled water in that order. The washed composite was lyophilized to prepare a composite of collagen/phosphophoryn (a sponge-like sheet). Also, collagen fibers to which phosphophoryn had not been added were thoroughly washed with distilled water, and then lyophilized to prepare a collagen composite (a sponge-like sheet) not containing phosphophoryn.

### Example 2: Experiment of implantation into rat femurs

### 1. Cell culture

The femurs of 8-week-old Fischer rats were excised, and cells in the bone marrow were extracted therefrom in accordance with a conventional technique. The extracted cells were cultured for 10 days under the following conditions. During the culture period, media were exchanged every 2 days, suspended hematocytes were removed, and osteoblasts adhering to the bottom were purified.

Culture conditions: temperature of 37°C; CO₂ level of 5%; α-MEM media (10% FBS + 1% glutamine + antibiotics)

The osteoblasts that had been cultured for 10 days in the manner described above were subcultured (10⁶ cells/ml), and cultured on collagen-phosphophoryn sheets (10 mm φ, 5 sheets) or collagen sheets (10 mm φ, 5 sheets) for 2 weeks under the following conditions.

Culture conditions: temperature of 37°C; CO₂ level of 5%; α-MEM media (10% FBS + 1% glutamine + antibiotics) + 10 mM β-glycerophosphate + 50 µg/ml of vitamin C phosphate + 10⁻⁸ M dexamethasone

The thus obtained sheets comprising the cultured cells were employed as samples for implantation.

### 2. Experiment of implantation

The femurs of 8-week-old Fischer rats (n = 5) were perforated using a drill (pore diameter of 2 mm). The prepared samples for implantation were cut into sizes that could be inserted into the perforations prepared above, and the cut samples were implanted in the perforated portions. Collagen sheets were implanted in the right legs of all rats, and collagen-phosphophoryn sheets were implanted in the left legs thereof. The femurs to which the collagen or collagen-phosphophoryn sheets had been implanted were excised 1, 2, 4, 6, and 8 weeks after implantation.

### 3. Observation of sites in which samples had been implanted

The rats that had reached the day determined for the excision of the implanted samples were anesthetized with 7% chloral hydrate (0.4 ml of chloral hydrate was intraperitoneally injected per 100 g of a rat's body weight). The pectoral regions of the anesthetized rats were opened, a fixing solution was injected through the heart (4% paraformaldehyde/0.25% glutaraldehyde), and the rats were perfusion-fixed (the time of perfusion: 15 min). After the completion of perfusion-fixation, femurs were excised from the rats. The excised femurs were defatted, demineralized, dehydrated with alcohol, and penetrated. The thus-processed femurs were embedded in paraffin in a manner such that the surfaces of the samples remained visible. The embedded samples were sliced into 5 µm-sections using a microtome, attached onto the slide glass, and spread on a paraffin-spreading apparatus. The spread paraffin sections were stained with hematoxylin and eosin and then observed under a microscope. The tissue images are shown in Fig. 1 and Fig. 2.

### 4. Results (Fig. 1 and Fig. 2)

Ossification became more advanced in the femurs in which collagen-phosphophoryn sheets had been implanted, in comparison with that in the femurs in which the collagen sheets had been implanted on the 1st week. The same was true on the second week and thereafter. Some ossification occurred when sheets consisting of collagen had been implanted, although ossification was found to be more advanced when collagen-phosphophoryn sheets had been implanted. Inflammation or other symptoms were not particularly observed.

### 5. Conclusion

Accordingly, the collagen-phosphophoryn sheet of the present invention was found to have higher biocompatibility and a better capacity for ossification than the collagen sheet. Since no inflammatory response was observed after implantation, the composite biomaterials of the present invention were found to be excellent in terms of safety.

All publications, patents, and patent applications cited herein are incorporated herein by reference in their entirety.

### Industrial Applicability

The present invention can provide novel composite biomaterials having excellent bioadaptability and osteoinductivity.

## Claims

1. Composite biomaterials comprising phosphophoryn and collagen.

2. The composite biomaterials according to claim 1, wherein the collagen is type I collagen.

3. The composite biomaterials according to claim 1 or 2, wherein the phosphophoryn is crosslinked to collagen.

4. The composite biomaterials according to any one of claims 1 to 3, which have sponge-like structures.

5. The composite biomaterials according to any one of claims 1 to 4, which further comprise at least one selected from among hydroxyapatite, β-TCP, α-TCP, polyglycolic acid, and polylactic acid.

6. The composite biomaterials according to any one of claims 1 to 5, which further comprise bone marrow-derived cells.

7. Artificial bones comprising the composite biomaterials according to any one of claims 1 to 6.

8. A method for producing composite biomaterials, wherein collagen fibers are crosslinked to phosphophoryn with divinylsulfone or 1-ethyl-3-(3-dimethylaminopropyl)carbodiamide, followed by lyophilization.
